Europäisches Patentamt

European Patent Office

Office européen des brevets

(19)

(11) Veröffentlichungsnummer: **0 276 392 B1**

(12) **EUROPÄISCHE PATENTSCHRIFT**

(45) Veröffentlichungstag der Patentschrift: **20.01.93**

(51) Int. Cl.5: **C07C 227/34**, C07C 323/58, C12P 13/04

(21) Anmeldenummer: **87116852.2**

(22) Anmeldetag: **14.11.87**

(54) **Verfahren zur Isolierung von L-Aminosäuren.**

(30) Priorität: **30.01.87 DE 3702689**

(43) Veröffentlichungstag der Anmeldung:
**03.08.88 Patentblatt 88/31**

(45) Bekanntmachung des Hinweises auf die
Patenterteilung:
**20.01.93 Patentblatt 93/03**

(84) Benannte Vertragsstaaten:
**BE CH DE FR GB LI NL SE**

(56) Entgegenhaltungen:
DE-A- 2 501 339
DE-A- 3 400 574
US-A- 3 386 888
US-A- 4 661 629

(73) Patentinhaber: **Degussa Aktiengesellschaft
Weissfrauenstrasse 9
W-6000 Frankfurt am Main 1(DE)**

(72) Erfinder: **Kleemann, Axel, Prof. Dr.
Bornweg 36
W-6052 Mühlheim 2(DE)**
Erfinder: **Klostermann, Kurt
Finkenweg 3
W-8752 Goldbach(DE)**
Erfinder: **Leuchtenberger, Wolfgang, Dr.
Geschwister-Scholl-Strasse 1
W-6454 Bruchköbel(DE)**
Erfinder: **Moerck, Rudi, Dr.
12 Cheyenne Drive
Montville NJ 07045(US)**
Erfinder: **Karrenbauer, Michael, Dr.
Hegaustrasse 1
W-7761 Moos-Bankholzen(DE)**

**Beschreibung**

Gegenstand der Erfindung ist ein Verfahren zur Isolierung von L-Aminosäuren aus den bei der Acylase-katalysierten Spaltung von N-Acetyl-D,L-aminosäuren anfallenden Spaltlösungen unter Verwendung eines stark sauren Ionenaustauschers in der $H^+$-Form.

Die Acylase-katalysierte Spaltung von N-Acetyl-D,L-aminosäuren in die L-Aminosäure, Essigsäure und N-Acetyl-D-aminosäure ist grundsätzlich bekannt. Die Spaltung wird üblicherweise in einer zwischen 0,4- und 1,0- molaren Lösung der N-Acetyl-D,L-aminosäure und bei einem pH-Wert zwischen 6 und 8 vorgenommen. Die N-Acetyl-D,L-aminosäure wird deshalb als Natrium- oder Kaliumsalz eingesetzt.

Es ist auch bekannt, die L-Aminosäure aus der Spaltlösung durch Eindampfen und anschließendes Abkühlen in kristalliner Form zu isolieren. Der Nachteil dieser direkten Aufarbeitung der rohen Spaltlösung liegt darin, daß zusammen mit der L-Aminosäure auch Natrium- oder Kaliumacetat ausfällt und die L-Aminosäure verunreinigt.

Die nach der Abtrennung der kristallisierten L-Aminosäure verbleibende Mutterlauge wird aus wirtschaftlichen und abwassertechnischen Gründen auf N-Acetyl-D,L-aminosäure aufgearbeitet. Bei der direkten Aufarbeitung der rohen Spaltlösung enthält die Mutterlauge unter anderem auch noch merkliche Mengen der L-Aminosäure. Soll diese Mutterlauge auf N-Acetyl-D,L-aminosäure aufgearbeitet werden, muß zunächst die enthaltene L-Aminosäure erneut acetyliert werden, weil sonst bei der Reracemisierung der N-Acetyl-D-aminosäure Peptide gebildet werden. Dies bedeutet, daß bereits durch Acylase in Freiheit gesetzte L-Aminosäure nach Acetylierung und Reracemisierung erneut der Acylase-katalysierten Spaltung unterworfen werden muß. Dadurch erhöht sich der Verbrauch an Acylase und es entsteht ein zusätzlicher Produktverlust bei der Reracemisierung.

Es ist schließlich auch bekannt, die in der rohen Spaltlösung enthaltene L-Aminosäure mit Hilfe eines stark sauren Ionenaustauschers in der $H^+$-Form zu isolieren. Dabei wird soviel Spaltlösung auf den Ionenaustauscher aufgegeben, daß dieser zu 80 bis 85% seiner Kapazität mit Alkalimetallionen und L-Aminosäuremolekülen beladen ist. Nach dem Auswaschen mit Wasser wird die L-Aminosäure mit einer wäßrigen Ammoniaklösung aus dem Ionenaustauscher eluiert. Aus dem Eluat wird durch Eindampfen und anschließendes Abkühlen die L-Aminosäure in kristalliner Form gewonnen. Wird zum Eluieren eine ca. 3N Ammoniaklösung verwendet, gelangen teilweise auch Alkalimetallionen in das Eluat und führen zu einem erhöhten Aschegehalt der kristallisierten L-Aminosäure. Diesem Nachteil kann zwar weitgehend abgeholfen werden, wenn eine verdünntere Ammoniaklösung, beispielsweise 0,4 oder 0,5N, eingesetzt wird. Dann erhält man aber ein sehr verdünntes Eluat, das eine verlängerte Eindampfzeit erfordert, was nun wieder zu einer verstärkten Nebenproduktbildung und/oder Racemisierung der L-Aminosäure führt. Ein weiterer Nachteil dieses bekannten Verfahrens besteht darin, daß die Kapazität des Ionenaustauschers nur zu etwa 45 bis 50% zur Bindung von Alkalimetallionen genutzt werden kann.

Das erfindungsgemäße Verfahren ist nun dadurch gekennzeichnet, daß der Ionenaustauscher bei einer Temperatur zwischen 30 und 90°C betrieben wird, daß auf ihn zunächst die Mutterlauge aus einem vorangegangenen Aufarbeitungszyklus, dann die Spaltlösung und schließlich Waschwasser aufgegeben wird, daß der Ablauf aus dem Ionenaustauscher ph-gesteuert in drei Fraktionen zerlegt wird, deren erste vom Beginn bis zum Erreichen eines pH-Werts von 2,0, deren zweite von dann bis zum nach vorübergehendem weiteren Absinken erneuten Erreichen eines pH-Werts von 2,0 und deren dritte von dann während der Aufgabe der Spaltlösung bis zum Erreichen eines pH-Werts von 5,0 aufgefangen wird, daß diese dritte Fraktion mit dem während der Aufgabe des Waschwassers anfallenden Ablauf vereinigt und die Mischung durch Zugabe von frischer Spaltlösung auf einen pH-Wert zwischen 4,0 und 6,0 eingestellt wird, daß aus der eingestellten Mischung in an sich bekannter Weise die L-Aminosäure durch Kristallisation isoliert wird, und daß die dabei anfallende Mutterlauge in einen folgenden Aufarbeitungszyklus zurückgeführt wird.

Das erfindungsgemäße Verfahren ist besonders geeignet für die Aufarbeitung der Spaltlösungen von N-Acetyl-D,L-methionin, N-Acetyl-D,L-valin und N-Acetyl-D,L-phenylalanin.

Geeignete Ionenaustauscher sind beispielsweise die handelsüblichen Produkte auf der Grundlage von sulfoniertem mit Divinylbenzol vernetztem Polystyrol.

Der Ionenaustauscher wird vorzugsweise bei einer Temperatur zwischen 60 und 80°C betrieben.

Auf den Ionenaustauscher wird zunächst die Mutterlauge aus der L-Aminosäure-Kristallisation eines vorangegangenen Aufarbeitungszyklus und dann die frische Spaltlösung, die einen pH-Wert zwischen 6,0 und 8,0 aufweist, aufgegeben. Zweckmäßigerweise werden beide Lösungen auf die gewünschte Temperatur vorgewärmt. Bei der erstmaligen Durchführung des erfindungsgemäßen Verfahrens, wenn noch keine Mutterlauge aus einem vorangegangenen Aufarbeitungszyklus vorliegt, wird gleich mit der Aufgabe der frischen Spaltlösung begonnen.

Der Ablauf aus dem Ionenaustauscher wird als eine erste Fraktion ("Abwasser-Fraktion") solange aufgefangen, bis der pH-Wert im Ablauf auf 2,0 absinkt. Diese Fraktion wird verworfen. Dann wird die Vorlage gewechselt und der nun folgende Ablauf wird als eine zweite Fraktion ("Acetyl-Fraktion") solange aufgefangen, bis der pH-Wert im Ablauf, der zunächst noch weiter absinkt, wieder auf 2,0 ansteigt. Diese Fraktion mit eine pH-Wert<2,0 enthält hauptsächlich die freie N-Acetyl-D-aminosäure und Essigsäure und wird deshalb der Reracemisierung zugeführt.

Die Vorlage wird erneut gewechselt und der nun folgende Ablauf wird als dritte Fraktion ("L-Aminosäure-Fraktion") aufgefangen. Während der Aufgabe der Mutterlauge und der Spaltlösung wird der Ionenaustauscher mit Alkalimetallionen und L-Aminosäuremolekülen beladen.

Infolge der unterschiedlichen Basizität der Alkalimetallionen und der L-Aminosäuremoleküle werden die Alkalmetallionen stärker an den Ionenaustauscher gebunden als die L-Aminosäuremoleküle. Nachkommende Alkalimetallionen verdrängen daher im Aufgabebereich des Ionenaustauschers bereits gebundene L-Aminosäuremoleküle in Richtung Ablaufbereich, wo sie wieder gebunden werden. In der Ionenaustauschersäule sind dann nach einiger Zeit im Aufgabebereich nur noch Alkalimetallionen und im Ablaufbereich nur noch L-Aminosäuremoleküle gebunden. Durch Aufgabe von weiterer Spaltlösung werden nun auch die zunächst noch in der Ionenaustauschersäule gebundenen L-Aminosäuremoleküle verdrängt und gelangen in den Ablauf, der außerdem noch restliche N-Acetyl-D-aminosäure und Essigsäure enthält. Die Aufgabe von Spaltlösung wird dann eingestellt, wenn der pH-Wert im Ablauf auf 5,0 angestiegen ist.

Der Ionenaustauscher wird nun solange mit Wasser gespült, bis sich keine gelösten Stoffe mehr im Ablauf befinden. Das Waschwasser wird zweckmäßigerweise ebenfalls auf die gewünschte Betriebstemperatur des Ionenaustauschers vorgewärmt. Der beim Spülen anfallende Ablauf wird mit der "L-Aminosäure-Fraktion" vereinigt.

Der Ionenaustauscher ist nun praktisch vollständig mit Alkalimetallionen beladen. Es wird somit eine maximale Ausnutzung seiner Kapazität erreicht. Er kann in an sich bekannter Weise mit einer verdünnten Säure, z.B. wäßriger Schwefelsäure, regeneriert werden.

Im Ablauf der Regenerierung kann keine L-Aminosäure mehr nachgewiesen werden.

Die Mischung aus der "L-Aminosäure-Fraktion" und dem Ablauf beim Spülen des Ionenaustauschers weist einen pH-Wert zwischen 2,5 und 3,5 auf. Dampft man diese Mischung direkt ein und kühlt sie dann zwecks Kristallisation ab, so ist die ausfallende L-Aminosäure stark mit N-Acetyl-D-aminosäure verunreinigt. Sie wird daher zunächst mit soviel frischer Spaltlösung versetzt, daß sich ein pH-Wert zwischen 4,0 und 6,0, vorzugsweise zwischen 4,5 und 5,5, ergibt. Wird nun diese Lösung unter vermindertem Druck auf etwa 20 bis etwa 50% des Ausgangsvolumens eingedampft und zwecks Kristallisation abgekühlt, gewinnt man eine L-Aminosäure, die nahezu frei von N-Acetyl-D-aminosäure ist und einen spezifikationsgerechten Aschegehalt und spezifischen Drehwert aufweist.

Die Isolierausbeute an L-Aminosäure liegt im allgemeinen bei 40 bis 80% der insgesamt, also in Form von Mutterlauge und frischer Spaltlösung, eingesetzten L-Aminosäure. Würde man die bei der L-Aminosäure-Kristallisation verbleibende Mutterlauge in die Reracemisierung und anschließende erneute Racematspaltung zurückführen, wäre dies mit unvermeidbaren Produktverlusten verbunden. Da aber die Mutterlauge in einen folgenden Aufarbeitungszyklus zurückgeführt wird, entstehen keine Produktverluste, sondern die Isolierausbeute an L-Aminosäure wird durch die ständige Zurückführung der Mutterlaugen auf über 95% gesteigert.

Die Erfindung wird durch die nachfolgenden Beispiele näher erläutert:

Beispiel 1:

Dieses Beispiel zeigt die erstmalige Durchführung des erfindungsgemäßen Verfahrens, wenn noch keine Mutterlauge aus einem vorherigen Aufarbeitungszyklus vorliegt, anhand einer Spaltlösung von N-Acetyl-D,L-methionin:

Verwendet wurde eine mit 1,5 l eines stark sauren Ionenaustauschers (Duolite C 26®) in der $H^+$-Form gefüllte Ionenaustauschersäule, die mittels heißen Wassers auf 70°C vorgewärmt wurde.

Aufgearbeitet wurde eine Spaltlösung mit folgenden Gehalten:

| | |
|---|---|
| 38,5 g/l | L-Methionin, |
| 66,3 g/l | N-Acetyl-D-methionin und |
| 14 g/l | Natriumionen. |

Diese Spaltlösung wurde mit einer Temperatur von 70°C und mit einer Durchflußgeschwindigkeit von 3,5 l/Stunde auf den vorbereiteten Ionenaustauscher aufgegeben, bis der pH-Wert im Ablauf auf 2,0 abgesunken war. Dann wurde die Vorlage gewechselt und die bis dahin angefallene erste Ablauf-Fraktion ("Abwasser-Fraktion") in einer Menge von 1,2 l wurde verworfen.

Bei der weiteren Aufgabe der Spaltlösung sank der pH-Wert im Ablauf zunächst unter 2,0 ab und stieg dann wieder an. Als der pH-Wert wieder 2,0 betrug, wurde erneut die Vorlage gewechselt. Die angefallene zweite Ablauf-Fraktion ("Acetyl-Fraktion") in einer Menge von 2,0 l kann der Reracemisierung zugeführt werden.

Bei der weiteren Aufgabe der Spaltlösung stieg der pH-Wert im Ablauf weiter an und die Aufgabe wurde solange fortgesetzt, bis ein pH-Wert von 5,0 erreicht war. Dies war nach der Aufgabe von insgesamt 4,8 l Spaltlösung der Fall. Nun wurde der Ionenaustauscher mit 2,0 l Wasser von 70°C nachgewaschen. Einschließlich des Waschwassers fiel eine dritte Ablauf-Fraktion ("L-Methionin-Fraktion") in einer Menge von 3,6 l und mit folgender Zusammensetzung an:

| 50 g/l | L-Methionin, |
|---|---|
| 51 g/l | N-Acetyl-D-methionin und |
| 7,7 g/l | Natriumionen. |

Diese "L-Methionin-Fraktion" wurde nun mit 3,0 l frischer Spaltlösung der oben bereits genannten Zusammensetzung vermischt. Die Mischung mit einem pH-Wert von 5,0 wurde unter vermindertem Druck bei einer Temperatur von 70°C eingedampft, bis L-Methionin auszufallen begann. Nun wurde die Suspension auf 80°C erwärmt und es wurde eine Aktivkohle-Klärung durchgeführt.

Das klare Filtrat wurde innerhalb von 7 Stunden auf 20°C abgekühlt. Das auskristallisierte L-Methionin wurde abfiltriert und getrocknet.

Ausbeute: 135 g (= 45% des in der verbrauchten Spaltlösung insgesamt enthaltenen L-Methionins).

Analysendaten:

| Gehalt: | 99,7% |
|---|---|
| Aschegehalt: | 0,02% |
| Transmission: | 98,5% |
| $[\alpha]_D^{20}$ : | + 25,5° |

Die zurückbleibende Mutterlauge hatte folgende Gehalte:

| 46 g/l | L-Methionin, |
|---|---|
| 98 g/l | N-Acetyl-D-methionin und |
| 20 g/l | Natriumionen |

Sie wird dem nächsten Aufarbeitungszyklus zugeführt.

Die Ionenaustauschersäule wurde von oben mit 3l einer 8 gewichtsprozentigen $H_2SO_4$ beaufschlagt, rückgespült und mit Wasser sulfatfrei gewaschen. Sie ist dann für die erneute Verwendung vorbereitet.

Beispiel 2:

Dieses Beispiel zeigt einen vollständigen Aufarbeitungszyklus nach dem erfindungsgemäßen Verfahren unter Mitverwendung der im Beispiel 1 angefallenen Mutterlauge:

Verwendet wurden die Ionenaustauschersäule und die Spaltlösung des Beispiels 1 und es wurde wiederum bei den Temperaturen des Beispiels 1 gearbeitet.

Auf den Ionenaustauscher wurden zunächst 3 l auf 70°C erwärmte Mutterlauge aus dem Beispiel 1 aufgegeben, anschließend 1,3 l auf 70°C erwärmte Spaltlösung und es wurde mit 2,2 l Wasser von 70°C nachgewaschen. Der Wechsel der Vorlage wurde jeweils bei Erreichen der im Beispiel 1 angegebenen pH-Werte vorgenommen. Es wurden folgende Fraktionen erhalten:

```
1. 700 ml "Abwasser-Fraktion"

2. 2,1 l  "Acetyl-Fraktion"

3. 3,7 l  "L-Methionin-Fraktion"

           mit 50 g/l L-Methionin,

               55 g/l N-Acetyl-D-methionin und

               9,5 g/l Natriumionen.
```

Die Aufarbeitung der "L-Methionin-Fraktion" erfolgte wie im Beispiel 1. Die Ausbeute an L-Methionin betrug 118 g (= 40%).

Analysendaten:

| Gehalt: | 99,8% |
|---|---|
| Aschegehalt: | 0,02% |
| Transmission: | 98% |
| $[\alpha]_D^{20}$ : | + 24,1° |

Bezogen auf den gesamten Gehalt an L-Methionin der in den Beispielen 1 und 2 eingesetzten Spaltlösung, beläuft sich die Isolierausbeute auf 54% L-Methionin. Unter Berücksichtigung des L-Methionins, welches sich in der Mutterlauge des Beispiels 2 befindet und in den nachfolgenden Aufarbeitungszyklen isoliert werden kann, liegt die Gesamtausbeute an kristallisiertem L-Methionin bei 95%.

Beispiel 3:

Verwendet wurde eine mit 30 l eines stark sauren Ionenaustauschers (Duolite C 26®) in der $H^+$-Form gefüllte Ionenaustauschersäule, die mittels heißem Wasser auf 70°C vorgewärmt wurde.

Auf den Ionenaustauscher wurden mit einer Durchflußgeschwindigkeit von 70 l/Stunde zunächst 63 l auf 70°C erwärmte Mutterlauge aus einer vorhergehenden L-Methionin-Kristallisation mit

| 36,8 g/l | L-Methionin |
|---|---|
| 110,5 g/l | Acetyl-D-methionin und |
| 19,8 g/l | Natriumionen |

aufgegeben, anschließend 21 l auf 70°C erwärmte Spaltlösung mit

| 37,0 g/l | L-Methionin |
|---|---|
| 67,4 g/l | N-Acetyl-D-methionin und |
| 14,6 g/l | Natriumionen |

und es wurde mit 40 l 70°C warmem Wasser nachgewaschen.

Der Wechsel der Vorlage wurde jeweils bei Erreichen der im Beispiel 1 angegebenen pH-Werte vorgenommen.

Es wurden folgende Fraktionen erhalten:

```
1.     19 l "Abwasser-Fraktion"

2.     42 l "Acetyl-Fraktion"

3.     63 l "L-Methionin-Fraktion"

          mit 47,7 g/l L-Methionin

              60,2 g/l Acetyl-D-methionin und

              7,5 g/l Natriumionen.
```

Die Aufarbeitung der "L-Methionin-Fraktion" erfolgte wie im Beispiel 1 unter Zusatz von 57 l frischer Spaltlösung der oben bereits angegebenen Zusammensetzung.

Analysendaten:

| Gehalt | 99,8 % |
|---|---|
| Aschegehalt | 0,05% |
| Transmission | 98 % |
| $[\alpha]_D^{20}$ | +24,3°. |

Beispiel 4:

Verwendet wurde die Ionenaustauschersäule des Beispiels 3 und es wurden wiederum auf 70°C vorgewärmte Mutterlauge, Spaltlösung und Wasser zum Nachwaschen eingesetzt.

Auf den Ionenaustauscher wurden zunächst 74 l Mutterlauge aus einer vorhergehenden L-Methionin-Kristallisation mit

| 41,8 g/l | L-Methionin |
|---|---|
| 119,8 g/l | Acetyl-D-methionin und |
| 20,8 g/l | Natriumionen |

aufgegeben, anschließend 10 l Spaltlösung mit

| 35,7 g/l | L-Methionin |
|---|---|
| 70,0 g/l | Acetyl-D-methionin und |
| 14,2 g/l | Natriumionen |

und es wurde mit 40 l Wasser nachgewaschen.

Der Wechsel der Vorlage wurde jeweils bei Erreichen der im Beispiel 1 angegebenen pH-Werte vorgenommen.

Es wurden folgende Fraktionen erhalten:

```
1. 14 l "Abwaser-Fraktion"

2. 47 l "Acetyl-Fraktion"

3. 63 l "L-Methionin-Fraktion"

          mit 54,3 g/l L-Methionin

              70,0 g/l Acetyl-D-methionin und

              7,0 g/l Natriumionen.
```

Die Aufarbeitung der "L-Methionin-Fraktion" erfolgte wie im Beispiel 1 unter Zusatz von 78,5 l frischer Spaltlösung der oben bereits angegebenen Zusammensetzung.

Die Ausbeute an L-Methionin betrug 2501 g (40% der Theorie).

Analysendaten:

| | |
|---|---|
| Gehalt | 98,5% |
| Aschegehalt | 0,3% |
| Transmission | 97,5 % |
| $[\alpha]_D^{20}$ | +24,1°. |

Beispiel 5:

Verwendet wurde eine mit 940 ml eines stark sauren Ionenaustauschers (Duolite C 26®) in der H[+]-Form gefüllte Ionenaustauschersäule, die mittels heißen Wassers auf 70°C vorgewärmt wurde.

Auf diese Säule wurden zunächst 990 ml der Mutterlauge aus einer vorhergehenden L-Valin-Kristallisation mit

| | |
|---|---|
| 24, 2 g/l | L-Valin, |
| 191 g/l | N-Acetyl-D-Valin und |
| 7,8 g/l | Natriumionen |

die auf 70°C erwärmt war, aufgegeben, anschließend 1 100 ml auf 70°C erwärmte Spaltlösung mit

| | |
|---|---|
| 31,8 g/l | L-Valin, |
| 51,6 g/l | N-Acetyl-D-valin und |
| 13,8 g/l | Natriumionen |

und es wurde mit 1 300 ml Wasser von 70°C nachgewaschen. Der Wechsel der Vorlage wurde jeweils bei Erreichen der im Beispiel 1 angegebenen pH-Werte vorgenommen. Es wurden folgende Fraktionen erhalten:

```
1. 650 ml "Abwasser-Fraktion"

2. 840 ml "Acetyl-Fraktion"

3. 1 900 ml "L-Valin-Fraktion"
              mit 30 g/l L-Valin,
                  61 g/l Acetyl-D-Valin und
              3,1 g/l Natriumionen.
```

Die Aufarbeitung der "L-Valin-Fraktion" erfolgte analog zum Beispiel 1 unter Zusatz von 1,8 l frischer Spaltlösung der oben bereits angegebenen Zusammensetzung. Die Ausbeute betrug 97 g L-Valin mit einem Gehalt > 99%. Dies entspricht einer Isolierausbeute von 83%.

Analysendaten:

EP 0 276 392 B1

| Aschegehalt: | 0,0% |
|---|---|
| Transmission: | 95,7% |
| $[\alpha]_D^{20}$ : | + 28,1° |

**Patentansprüche**

1. Verfahren zur Isolierung von L-Aminosäuren aus den bei der Acylase-katalysierten Spaltung von N-Acetyl-D,L-aminosäuren anfallenden Spaltlösungen unter Verwendung eines stark sauren Ionenaustauschers in der H$^+$-Form, dadurch gekennzeichnet, daß der Ionen austauscher bei einer Temperatur zwischen 30 und 90°C betrieben wird, daß auf ihn zunächst die Mutterlauge aus einem vorangegangenen Aufarbeitungszyklus, dann die Spaltlösung und schließlich Waschwasser aufgegeben wird, daß der Ablauf aus dem Ionenaustauscher pH-gesteuert in drei Fraktionen zerlegt wird, deren erste vom Beginn bis zum Erreichen eines pH-Werts von 2,0, deren zweite von dann bis zum nach vorübergehendem weiteren Absinken erneuten Erreichen eines pH-Werts von 2,0 und deren dritte von dann während der Aufgabe der Spaltlösung bis zum Erreichen eines pH-Werts von 5,0 aufgefangen wird, daß diese dritte Fraktion mit dem während der Aufgabe des Waschwassers anfallenden Ablauf vereinigt und die Mischung durch Zugabe von frischer Spaltlösung auf einen pH-Wert zwischen 4,0 und 6,0 eingestellt wird, daß aus der eingestellten Mischung in an sich bekannter Weise die L-Aminosäure durch Kristallisation isoliert wird, und daß die dabei anfallende Mutterlauge in einen folgenden Aufarbeitungszyklus zurückgeführt wird.

2. Verfahren nach Anspruch 1, dadurch gekennzeichnet, daß der Ionenaustauscher bei einer Temperatur zwischen 60 und 80°C betrieben wird.

3. Verfahren nach Anspruch 1 oder 2, dadurch gekennzeichnet, daß die Mischung aus der dritten Fraktion und dem während der Aufgabe des Waschwassers anfallenden Ablauf durch Zugabe von frischer Spaltlösung auf einen pH-Wert zwischen 4,5 und 5,5 eingestellt wird.

**Claims**

1. A process for the isolation of L-amino acids from the separation solutions accumulating in the acylase-catalyzed separation of n-acetyl-D,L-amino acids using a strongly acidic ion exchanger in the H$^+$ form, characterized in that the ion exchanger is operated at a temperature of 30 to 90°C, in that first the mother liquor from a preceding working-up cycle, then the separation solution and, finally, washing water are applied to the ion exchanger, in that the effluent from the ion exchanger is divided under pH control into three fractions of which the first is collected from the beginning until a pH value of 2.0 is reached, of which the second is collected from then until a pH value of 2.0 is again reached after a further temporary fall and of which the third is collected from then during the application of the separation solution until a pH value of 5.0 is reached in that this third fraction is combined with the effluent accumulating during application of the washing water and the mixture is regulated to a pH value of 4.0 to 6.0 by addition of fresh separation solution, in that the L-amino acid is isolated from the pH regulated mixture in known manner by crystallization and in that the mother liquor accumulating is returned to a following working-up cycle.

2. A process as claimed in claim 1, characterized in that the ion exchanger is operated at a temperature of 60 to 80°C.

3. A process as claimed in claim 1 or 2, characterized in that the mixture of the third fraction and the effluent accumulating during application of the washing water is regulated to a pH value of 4.5 to 5.5 by addition of fresh separation solution.

**Revendications**

1. Procédé d'isolement d'amino-acides (L) provenant des solutions de coupure qui se produisent dans la coupure catalysée par une acylase, des acides aminés N-acétylés (DL), par utilisation d'un échangeur d'ions fortement acide sous la forme H$^+$, procédé d'isolement, caractérisé en ce que l'échangeur d'ions est mis en fonctionnement à une température comprise entre 30° et 90°C, que l'on verse sur celui-ci en premier lieu les eaux-mères provenant d'un cycle d'épuration précédent, puis la solution de coupure et enfin de l'eau de lavage, que l'écoulement à la sortie de l'échangeur d'ions est divisé sous le contrôle du pH en trois fractions dont la première est recueillie du début jusqu'à l'obtention d'une valeur de pH de 2,0, dont la deuxième est recueillie à partir de là jusqu'à l'obtention renouvelée d'une valeur de pH de 2,0 après un autre abaissement transitoire et dont la troisième est recueillie à partir de là pendant que l'on verse la solution de coupure jusqu'à obtention d'une valeur de pH de 5,0, que l'on réunit cette troisième fraction avec l'écoulement qui se produit pendant que l'on verse l'eau de lavage et le mélange est ajusté par addition de solution de coupure fraîche, à une valeur de pH comprise entre 4,0 et 6,0, que l'on isole les amino-acides L par cristallisation à partir du mélange ajusté d'une manière connue en soi et que les eauxmères ainsi produites sont ramenées dans un cycle suivant de purification.

2. Procédé selon la revendication 1, caractérisé en ce que l'échangeur d'ions est mis en fonctionnement à une température allant de 60 à 80°C.

3. Procédé selon les revendications 1 ou 2, caractérisé en ce que le mélange provenant de la troisième fraction et de l'écoulement qui se produit pendant que l'on verse des eaux de lavage, est ajusté à une valeur de pH comprise entre 4,5 et 5,5 par addition de solution de coupure fraîche.